# FASCICULE DE BREVET EUROPEEN

(11) **EP 0 950 866 B1**
(45) Date de publication et mention de la délivrance du brevet: **14.06.2006**
(21) Numéro de dépôt: 98400911.8
(22) Date de dépôt: 14.04.1998
(51) Int. Cl.: F24F 3/16, B01L 1/04, C12M 1/00

(54) **Procédé de réalisation de cultures cellulaires**
Verfahren zur Herstellung von Zellkulturen
Process for preparing cell cultures

(43) Date de publication de la demande: 20.10.1999
(73) Titulaire: Laboratoires Genevrier, 06600 Antibes (FR)
(72) Inventeur: Vacher, Dominique, 06780 Saint-Cezaire-Sur-Siagne (FR)
(74) Mandataire: Burtin, Jean-François

(56) Documents cités:
- WO-A-81/01455
- WO-A-92/12631
- DE-A- 3 925 941
- FR-A- 2 605 901
- US-A- 5 259 812
- US-A- 5 545 086

## Description

La présente invention se rapporte à un procédé de réalisation de cultures cellulaires.

Elle a plus particulièrement pour objet un procédé tel que défini aux revendications.

Ce dispositif peut être complété par un ou plusieurs laboratoires de mise au point ou de développement.

La cascade de pressions est obtenue par circulation d'air turbulent stérile qui s'effectue par soufflage à travers plusieurs types de filtres stérilisants comme les filtres HEPA et par des diffuseurs disposés au plafond.

La communication entre les différents locaux se fait par l'intermédiaire de guichets ou passe-plats. Ceux-ci sont ventilés en air stérile et assurent une sécurité supplémentaire entre la salle blanche assurant les cultures primaires après la biopsie et les salles blanches adjacentes fonctionnant en surpression.

Selon l'invention l'implantation des locaux se fait selon une cascade de pressions allant des sas d'entrée en surpression vers les locaux de mise en culture de cellules primaires pour aboutir aux locaux où s'effectuent les cultures de cellules secondaires. Les pressions utilisées vont de 5 pascals à 45 pascals. Il n'existe entre les locaux accolés, de communication intérieure que par l'intermédiaire de passe-plats. Les locaux sont équipés également d'un sas d'entrée et de sortie pour le matériel en surpression, d'un sas de sortie en surpression, permettant l'évacuation des déchets. Tous les sas d'entrée ou les sas de sortie débouchent sur le couloir de circulation.

Dans un mode du procédé selon l'invention, particulièrement préféré, l'ensemble des locaux présente une configuration en forme de rectangle allongé comportant successivement une salle de traitement des biopsies et de culture primaire, placée en dépression comportant un sas d'entrée en surpression, un sas de sortie pour le personnel en dépression et un sas d'entrée pour le matériel et un sas de sortie pour les déchets, les deux en surpression, un ou plusieurs passe-plats de communication ventilés puis- accolée à ladite salle -une salle destinée à la préparation des milieux et des réactifs de culture spécialisés, à température constante, comportant un sas d'entrée pour le personnel, en surpression et un sas d'entrée pour le matériel également en surpression, et un sas pour le matériel et un sas pour l'évacuation des déchets, alors que la salle de préparation concernée est placée sous une pression plus élevée ; une salle de contrôle destinée à assurer la validation des méthodes et la qualité des produits, cette salle étant en surpression moyenne et les sas d'entrée étant eux-mêmes placés sous légère surpression.

Chaque laboratoire comporte donc un ou des sas d'entrée pour le personnel et pour le matériel neuf et un ou des sas de sortie permettant l'évacuation du matériel usagé.

A cette salle est accolée une pièce destinée à effectuer la production des cellules pouvant entrer en co-culture. Cette salle est la seule à communiquer par l'intérieur à une salle adjacente où on peut effectuer une irradiation de ces cellules si nécessaire. Elle peut comporter des dispositifs de communication avec toutes les pièces adjacentes du type passe-plats.

L'implantation comporte encore accolée par un côté, une salle de cryogénie permettant la congélation et la cryoconservation des échantillons de cellules irradiés à la température aussi basse que celle de l'azote liquide, sous forme d'un courant d'azote liquide.

L'implantation comporte encore une salle de cultures secondaires correspondant au produit fini en forte surpression, relié par l'intérieur par un passe-plat ventilé assurant la communication avec un ou des laboratoires de cultures primaires.

L'ensemble des pièces formant une telle implantation débouche sur un vestibule qui conduit au couloir de circulation périphérique.

Plus spécifiquement l'invention concerne l'implantation d'un plateau technique destiné à assurer la mise en culture de fragments de peau qui comprend un atelier où l'on traite les biopsies cutanées de cellules de la peau et en particulier des Kératinocytes (A), un atelier accolé où on effectue la préparation et l'incubation des milieux et réactifs de culture, appropriés pour ces fragments de cellules de peau (B) pendant la période appropriée pour avoir une production maximale, un laboratoire adjacent où s'effectuera la validation des produits et le contrôle interne de la qualité des produits (C), peut s'effectuer l'irradiation (D) par les rayons gamma des fibroblastes pour les rendre post-mitotique comme une salle de production de cultures de fibroblastes 3T3 (E) utilisés comme assurant le développement des Kératinocytes, une salle de cryogénie (F) comportant un congélateur à -80° C et un bac à azote liquide pour cryoconserver les Kératinocytes et les fibroblastes support, de manière à congeler essentiellement les Kératinocytes humains et ainsi à assurer la traçabilité des Kératinocytes pour pouvoir revenir ensuite au patient d'origine de manière à réaliser une Masterbank de fibroblastes. On peut se garder la possibilité de greffes autologues dépiderme.

Les produits congelés passent ensuite dans une salle de cultures secondaires (G) accolée à la salle de cultures primaires (A) accolée par un côté, dans laquelle on effectue le développement des Kératinocytes pour obtenir des cultures secondaires que l'on répartit dans des bacs ou des barquettes à double emballage, stériles, en polyéthylène (PETG) operculés.

Enfin l'implantation comporte accolé à la salle de traitement de la biopsie (A) et de cultures primaires, un laboratoire de développement (I) qui assure l'emballage final, avec lequel communique une salle de désinfection par autoclavage (J), destiné à assurer la production d'eau stérile, des outils et des matériaux de traitement de la biopsie. La pièce d'autoclavage communique avec le laboratoire de développement (I) par un passage interne. Cette pièce comporte également un sas d'entrée et de sortie pour le matériel (K) et un sas de sortie pour les déchets.

Le laboratoire de développement et la pièce d'autoclavage fonctionnent à la pression atmosphérique.

Selon une modalité particulière de l'implantation selon l'invention
⇒ les locaux sont ventilés par une soufflerie permettant les taux de renouvellement compris entre 30 et 50 par heure à l'aide de deux centrales d'air comportant trois étages de filtration et laissant passer 20 % d'air neuf à chaque impulsion,
⇒ la salle blanche (A) l'atelier de biopsie et de culture primaire fonctionnent en dépression et le reste de la plate-forme technique fonctionne en surpression,
⇒ le soufflage d'air turbulent stérile s'effectue à travers des filtres HEPA par l'intermédiaire de diffuseurs disposés au plafond des salles,
⇒ un dispositif permet la reprise de l'air en position basse à travers des cloisons d'extraction,
⇒ on dispose une cascade de pressions entre les salles blanches stériles d'une part, et entre le couloir de circulation et les salles blanches d'autre part, s'échelonnant de 5 pascals (pression faible) à 45 pascals (forte surpression),
⇒ les passe-plats de communication sont non ventilés ou ventilés entre la salle blanche de biopsie et les salles blanches accolées,
⇒ pour chaque salle blanche on dispose un sas d'entrée pour les personnes, ventilé,
⇒ les sas d'entrée du matériel sont ventilés et le sas de sortie -notamment pour l'évacuation des déchets sont ventilés dans chaque salle blanche,
⇒ les portes des sas de la salle blanche (A) de biopsie comportent des verrous électromagnétiques de sécurité,
⇒ les portes des sas des autres salles blanches sont du type à contact de feuillure relié à un affichage lumineux interne et externe, comme par exemple une lumière rouge et une lumière verte.

Les différentes salles blanches comportent des paillasses en matériau non poreux, plus facilement lavable, du type CORIANO et qui épousent les formes et les obstacles qu'elles contournent.

Le plateau technique ainsi constitué, a notamment pour objet non seulement la culture de cellules de la peau et en particulier de Kératinocytes prélevés sur des sujets en vue de la réparation de surfaces lésées, contusionnées ou brûlées mais aussi l'étude et le développement d'autres procédés de thérapie cellulaire chez l'homme, pouvant faire intervenir tout type de cellules humaines.

Le plateau technique mis en oeuvre dans le procédé selon l'invention comporte également différentes zones en atmosphère non contrôlée destinées à assurer la conservation et le stockage de l'ensemble des produits et des matériaux qui ne nécessitent pas de précautions particulières.

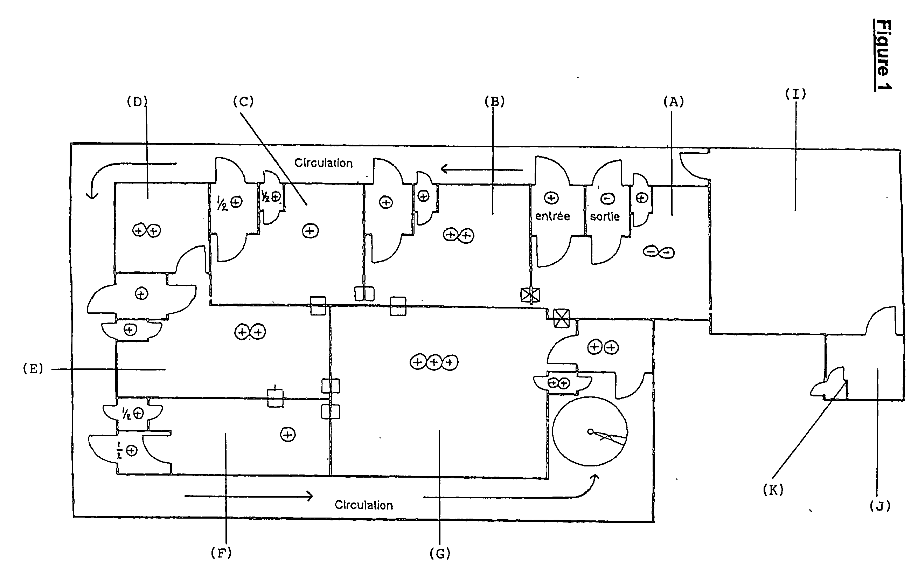

## Revendications

1. Procédé de réalisation de cultures cellulaires qui consiste à mettre en oeuvre un plateau technique comportant un ou plusieurs laboratoires de contrôle, un ou plusieurs laboratoire de développement et/ou des laboratoires de mise au point, dans lesquels tous les laboratoires accolés les uns aux autres sont entourés d'un couloir de circulation communiquant avec lesdits laboratoires par l'intermédiaire de sas en surpression, le plateaur comportant des pièces en atmosphère contrôlée dites salles blanches, comportant d'une part au moins un laboratoire de traitement du prélèvement et de mise en culture primaire des cellules, et d'autrepart un atelier immédiatement adjacent dans lequel on effectue la préparation et la mise en incubation des milieux et réactifs de culture, un ou et plusieurs ateliers où l'on contrôle les produits fabriqués, une salle de cryogémie et un ou plusieurs ateliers de mise en culture secondaire des cellules et un ou plusieurs laboratoires de mise au point et de développement, l'ensemble des locaux étant disposé de telle sorte que l'entrée ou la sortie du matériel et/ou du personnel de chaque local s'effectue par l'intermédiaire de sas en surpression obtenue par soufflage d'air filtré, stérile, réalisant une cascade de pressions entre, d'une part, le laboratoire de mise en culture primaire qui fonctionne en dépression, et, d'autre part, les autres salles dites blanches, par l'intermédiaire du couloir de circulation disposé à la périphérie où règne la pression atmosphérique.

2. Procédé de réalisation de cultures cellulaires selon la revendication 1, consiste à mettre en oeuvre un plateau technique dans lequel l'ensemble des locaux présente une configuration en forme de rectangle allongé comportant successivement une salle de traitement de la biopsie cutanée, placée en dépression, un sas d'entrée en surpression, un sas de sortie pour le personnel, en dépression et un sas d'entrée pour le matériel en surpression, puis accolée à elle une salle destinée à la préparation et à la mise en culture de milieux et réactifs de culture spécialisés, comportant des sas d'entrée en surpression, une salle de contrôle destinée à assurer la validation des méthodes et de la qualité des produits, une salle de production de fibroblastes qui entrent en co-culture avec les Kératinocytes, et éventuellement une salle pourvue d'un irradiateur, une salle de cryogénie permettant la congélation et la conservation des échantillons, une salle de préparation des cultures secondaires, toutes ces pièces étant en communication avec du couloir de circulation placé à la pression atmosphérique.

3. Procédé de réalisation de cultures cellulaires par mise en oeuvre d'un plateau technique selon la revendication 1, **caractérisé en ce que** l'ensemble des pièces formant une telle implantation, débouche sur un vestibule qui conduit au couloir de circulation périphérique.

4. Procédé de réalisation de cultures cellulaires selon la revendication 1, **caractérisé en ce que** la pièce de prélèvement sert à traiter des fragments de peau prélevés par biopsie pour réaliser la culture de Kératinocytes.

5. Procédé de réalisation de cultures cellulaires selon la revendication 1, **caractérisé en ce que** la salle d'irradiation est conçue de façon à assurer l'irradiation des cultures cellulaires par les rayons gamma.

6. Procédé de réalisation de cultures cellulaires selon la revendication 1, **caractérisé en ce que** la salle de production des fibroblastes irradiés permet d'assurer la mise en culture de fibroblastes en tant que couche nourricière des Kératinocytes.

7. Procédé de réalisation de cultures cellulaires selon la revendication 1, **caractérisé en ce que** la salle de cryogénie assure la congélation et la conservation des cultures de cellules à l'aide d'un congélateur à -70° C et d'un cryoconservateur à azote liquide.

8. Procédé de réalisation de cultures cellulaires selon la revendication 1, qui comporte également une salle de cultures secondaires dans laquelle on effectue le développement des Kératinocytes après amplification en cultures primaires voire secondaire que l'on répartit dans des bacs ou des barquettes à double emballage, stériles, en polyéthylène ou operculées, dans le but d'obtenir des greffons d'épiderme cultivés.

9. Procédé de réalisation de cultures cellulaires selon la revendication 1, qui comporte en outre l'implantation d'un ou plusieurs laboratoire de développement qui assure également le conditionnement final et l'emballage des produits.

## Claims

1. A method for the realization of cell cultures, consisting in implementing a technical support center including one or several control laboratories, one or several development laboratories and/or settlement laboratories, in which all the laboratories, next to each others, are surrounded by an aisle communicating with the said laboratories through pressurized locks, the support center including controlled atmosphere rooms called "white rooms", comporting on one hand a laboratory for the treatment of the sample and primary growth of the cells, and on the other hand an immediate neighbour laboratory for the preparation and incubation of the culture mediums and reagents, one or more rooms for the control of manufactured items, one cryogenics room, one or more rooms for the secondary growth of the cells and one or more settlement and development laboratories, the whole of the premises being settled so that the entrance or leaving of material and/or people of each room occurs through pressurized locks, the overpressure being obtained by filtered, sterile air blast, realizing a pressure cascade between, on one hand, the primary growth laboratory, working under vacuum, and on the other hand, the other "white rooms", through the external aisle at the atmospheric pressure.

2. A method for the realization of cell cultures according to claim 1, consisting in implementing a technical support center in which the whole of the premises is shaped as a long rectangle including successively a snipe-test room, working under vacuum, a pressurized entrance airlock, a staff leaving airlock under vacuum and a pressurized furniture entrance, and next to it a room for the preparation and incubation of the culture mediums and reagents, including pressurized entrance locks, a control room for the validation of the methods and of the quality of the products, a room for the production of fibroblasts used in the co-culture with keratinocytes, and optionally an irradiator room, a cryogenics room for the freezing and keeping of the samples, a room for the preparation of the secondary cultures, all these room communicating with the aisle at the atmospheric pressure.

3. A method for the realization of cell cultures by implementing a technical support center according to claim 1, **characterized in that** the whole of the rooms forming such an establishment open onto a hallway leading to the external aisle.

4. A method for the realization of cell cultures according to claim 1, **characterized in that** the laboratory for the treatment of the sample is used for the treatment of pieces of skin sampled by biopsy to realize the culture of keratinocytes.

5. A method for the realization of cell cultures according to claim 1, **characterized in that** the irradiator room is set to irradiate the cell cultures by gamma-ray.

6. A method for the realization of cell cultures according to claim 1, **characterized in that** the room for the production of irradiated fibroblasts allows the growth of fibroblasts as feeding layer for the keratinocytes.

7. A method for the realization of cell cultures according to claim 1, **characterized in that** the cryogenics room allows the freezing and keeping of the cell cultures by means of a -70 °C freezer and a liquid nitrogen refrigerator.

8. A method for the realization of cell cultures according to claim 1, also including a room for the secondary cultures, in which the keratinocytes are grown after primary or secondary culture amplification, shared out in polyethylene or capped, sterile, double-envelope tubs or trays, to obtain cultivated epidermis grafts.

9. A method for the realization of cell cultures according to claim 1, also including the settling of one or more development laboratories, also in charge with the final conditioning and the packaging of the products.

## Patentansprüche

1. Verfahren für die Ausführung zellularen Zuchten, das besteht darin man eine technische Bühne Gebrauch macht, die eine oder mehrere Kontrollaboratorien, eine oder mehrere Entwicklungslaboratorien, eine oder mehrere Klarstellungslaboratorien, enthält, worin alle die miteinender verbundene Laboratorien mit einem Umfangdurchgang umgegeben sind, der mit diesen Laboratorien über einem unter Überdrück angestellte Schleusekammer, in Verbindung ist der Buhne Zimmer unter regulierte Atmosphäre, die Zimmer die sogenannt "weiße Zimmer" sind, einhaltende einerseits zumindest eine Laboratorium für die Behandlung der Probe und für die primäre Zucht der Zellen und anderseits ein unmittelbare angrenzende Werkstatt, worin die Bereitung und die Bebrütung der Media und der Zuchtreagenzien, ausgeführt werden, ein oder mehrere Werkstätte wo die Erzeugnisse Kontrolliert werden, eine Kryogenie Zimmer, eine oder mehrere Werkstätte für die sekundären Zucht der Zellen, und eine oder mehrere Laboratorien für die Klarstellung und die Entwicklung, einschließt wo die ganze Zimmer so angestellt sind, daß der Eintritt oder Ausgang des Materials und/oder der Mannschaft jede Zimmer, durch Schleusekammer die unter Überdruck angeordnet sind, die durch Blasung von filtrierte, keimfreie Luft erzeugen wird, der eine Druckkaskade zwischen einerseits das Laboratorium für primären Zucht der unter Unterdruck funktioniert und anderseits die andere Zimmer, die "weiße Zimmer" genannt sind, durch dem Umfangskorridor der an der Peripherie angeordnet ist, wo die atmosphärische Drück herrscht.

2. Verfahren für die Ausführung der zellularen Zuchten nach Anspruch 1, das besteht darin man eine technische Bühne Gebrauch macht, wo die Gesamheit der Zimmer die Gestaltung eine ausgedehnte Rechtecke hat, die nach und nach eine Zimmer für die Behandlung der Biopsie der Haut, der in Unterdruck angeordnet ist, eine unter Überdrück angeordnete Schleusezimmer für den Eingang, eine Schleusezimmer für der Ausgang der Mannschaft, die unter Unterdruck angeordnet ist, und eine Schleusekammer für das Eingang des Materials, die unter Überdrück angeordnet ist, dann an diese anschließende, eine Zimmer für die Herstellung und die Zuchtung der Media, und der spezialisierte Reagenzien für die Züchtung die Schleusekammer für den Eingang umfäßt, die unter Überdrück angeordnet sind, eine Kontrollzimmer, die für der Gultigkeiterklärung der Methoden und der Qualität der Erzeugnisse besorgen bestimmt ist, eine Zimmer für die Herstellung der Fibroblasten, die mit der Keratinocyten in Mitzucht eintreten, und gegebenenfalls eine mit einem Strahlungsgerät vorgesehene Zimmer, eine Kryogenie Zimmer, die das Gefrierung und die Bewährung der Probe erlaubt, eine Zimmer für die Bereitung der sekundären Züchten, alle diese Zimmer damit der Umlaufsdurchgang verbunden sind, auf die atmosphärischen Druck angeordneten sind.

3. Verfahren zur Ausführung Zellularen Zuchten durch die Gebrauchmachung einer technische Bühne, nach Anspruch 1, **dadurch gekennzeichnet, daß** die Gesamtheit der Zimmer, die eine solche Einführung bilden, in einem Hausflur mundet, der zum der periphärischen Umgang leitet.

4. Verfahren zur Ausführung Zellularen Zuchten nach Anspruch 1, **dadurch gekennzeichnet, daß** die Entnahme Zimmer für die Behandlung von Bruchstücke von Haut dient, die durch Biopsie entnehmen werden um die Kultur von Keratinozyten zu durchführen.

5. Verfahren zur Durchführung zellularen Zuchten Anspruch 1, **dadurch gekennzeichnet, daß** die Bestrahlungszimmer ausgedacht wird, um die Bestrahlung der zellulären Züchte durch die Gamma Strahlen zu besichtigen.

6. Verfahren zum Ausführung zellularen Zuchten nach Anspruch 1, **dadurch gekennzeichnet, daß** die Herstellungszimmer für die bestrahlte Fibroblasten besorgt, die Zucht der Fibroblasten als ernährende schicht der Keratinozyten erlaubt.

7. Verfahren zur Ausführung zellularen Zuchten nach Anspruch 1, **dadurch gekennzeichnet, daß** die Zimmer für Kryogenie die Gefrierung und die Bewahrung der Zuchten von Zellen mittels einer um -70°C Gefrierungsgerät und mittels einem Kryobewahrer mit flüssigen Stickstoff erlaubt.

8. Verfahren für Ausführung von zellularen Zuchten nach Anspruch 1, das auch eine Zimmer für die sekundären Zuchten enthält, in dem man der Entwicklung der Keratinozyten nach Vergrößerung in primären sogar sekundären Zuchten durchführt, die in mit Doppel Verpackung vorgesehene keimfreie aus Polyathylän oder mit einem Deckel aus gerüstete Schale oder Gefäße, um die kultivierte Transplantaten von Oberhaut zu erhalten.

9. Verfahren zur Ausführung zellularen Züchten nach Anspruch 1, das zudem die Einführung einer order mehreren Entwicklungslaboratorien beinhalten, die auch die endgültige Konditionierung und die Verpackung der Produkten erlaubt.
